(19) Europäisches Patentamt
European Patent Office
Office européen des brevets

(11) Publication number: 0 299 975 B1

# (12) EUROPEAN PATENT SPECIFICATION

(45) Date of publication of patent specification: 11.11.92

(51) Int. Cl.5: A61K 31/70, A61K 31/56, A61K 31/725

(21) Application number: 87902995.7

(22) Date of filing: 03.04.87

(86) International application number: PCT/US87/00704

(87) International publication number: WO 87/05808 (08.10.87 87/22)

## (54) COMPOSITION FOR ARRESTING ANGIOGENESIS AND CAPILLARY, CELL OR MEMBRANE LEAKAGE.

(30) Priority: 04.04.86 US 848288
22.05.86 US 865981
02.03.87 US 20859

(43) Date of publication of application: 25.01.89 Bulletin 89/04

(45) Publication of the grant of the patent: 11.11.92 Bulletin 92/46

(84) Designated Contracting States: AT BE CH DE FR GB IT LI LU NL SE

(56) References cited:
EP-A- 114 589

THERAPIEWOCHE, vol. 33, July 1983, Verlag G. Braun,Karlsruhe (DE); E.SCHACHT et al., pp. 3756-3760.

JOURNAL OF UROLOGY, vol. 135, no. 4, part 2, May 1986, Baltimore, MD (US); L.GILLESPIE et al., p. 188a, no. 337.

(73) Proprietor: Angiogenics Limited
Suite 210 120 South Spalding Drive
Beverly Hills, CA 90212(US)

(72) Inventor: GILLESPIE, Larrian
120 South Spalding Drive, Suite 210
Beverly Hills, CA 90212(US)

(74) Representative: Lamb, John Baxter et al
MARKS & CLERK 57-60 Lincoln's Inn Fields
London WC2A 3LS(GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 299 975 B1

SCIENCE, vol. 221, no. 4612, August 1983, Washington, DC (US); J.FOLKMAN et al., pp. 719-725.

JOURNAL OF CELL BIOLOGY, abstract 581, October 1984, US; CRUM et al., p. 158a.

SCIENCE, vol. 230, no. 4732, December 1985, Washington, DC (US); CRUM et al., pp. 1375-1378.

PROCEEDINGS OF THE NATL. ACADEMY OF SCIENCE USA, vol. 78, no. 2, February 1981, US; J.CROSS et al., pp. 1176-1180.

## Description

The present invention relates to novel compositions for oral administration, useful for arresting angiogenesis and membrane, cell or capillary leakage, such as in abnormal angiogenesis, e.g. abnormal capillary growth, when used in effective amounts.

Steroids generally have constituted a standard treatment when administered topically, percutaneously and intravenously by virtue of their anti-inflammatory effects.

Heparin and heparin fragments, when used alone have been reported to enhance tumor angiogenesis ("Angiogenesis Inhibition and Tumor Regression Caused by Heparin or a Heparin Fragment in the Presence of Cortisone", by Folkman et al. Science (1983) 221:719-725. The same article reports that angiogenesis can be inhibited by the combination of heparin and cortisone based upon animal tests. No human tests were conducted.

European Patent No. 0 114 589, published August 1, 1984, in the name of FOLKMAN et al., as well as the above article disclose a technique for the inhibition of angiogenesis in mammals by treatment with heparin and specific heparin fragments, in combination with a member selected from cortisone, hydrocortisone or the 11-alpha isomer of hydrocortisone so as to inhibit angiogenesis with subsequent regression of large tumor masses and alleged prevention of tumor metastasis in mammals.

In these materials angiogenesis is defined as the growth of new capillary blood vessels which is important in normal tissue growth such as the development of the embryo, formation of the corpus luteum and wound healing. It is also stated to be a component in pathologic processes such as chronic inflammation, certain immune responses and neoplasia. Angiogenesis is stated to be a property of most solid tumors and is necessary for their continued growth.

The patent application reports that heparin alone enhances the intensity of angiogenesis induced by tumors in vivo. Reference is made to work by SHUBIK et al. (J. Natl. Cancer Inst., Vol. 57, 769-774, [1976]) in which 6 alpha-methyl-prednisolone partially suppressed tumor angiogenesis in hamster cheek pouch under certain conditions. Other publications have reported continued growth of tumors even in the presence of large doses of cortisone.

The application goes on to state that heparin fragments which are hexasaccharide or larger together with cortisone or hydrocortisone or the 11-alpha isomer of hydrocortisone will inhibit angiogenesis in mammals. The effect on angiogenesis is more definitively discussed in an article entitled: Toward a New Understanding of Vascular Proliferative Disease in Children, Pediatrics, Vol. 74, No. 5, November 1984.

The use of the purified form of heparin and heparin fragment in combination with certain steroid is also suggested by Crum et al., J. Cell Biology, October 1984, Abstract No. 581, page 158a.

The patent application is silent as to a key aspect of the mechanism behind angiogenesis, namely capillary exchange or leakage which Applicant alone has recognized to be the fundamental mechanism underlying angiogenesis.

In another approach, the use of sodium pentosan polysulfate $SP_{54}$, as an alternative to heparin, in the treatment of interstitial cystitis is disclosed (Successful Treatment of Interstitial Cystitis with Sodium Pentosanpolysulfate, by C. Lowell Parsons et al., Journal of Urology. pp. 51-53, 1983). The authors indicate that $SP_{54}$ is safe in humans, but mistakenly conclude that the method of action of the compound was through excretion in urine, and the relationship between the disease and its treatment to angiogenesis is not appreciated. This has now been found to be erroneous because the fragment is destroyed in the kidney. The lower anticoagulant activity of the compound in comparison to heparin is also disclosed, see also Marsh, N. Gaffney, P.J.: The Effect of Pentosan Polysulfate ($SP_{54}$) on the Fibrinolytic System of Man, IX Int. Congress on Thrombosis and Haemostasis, Stockholm, 1983.

The principles underlying the efficacy of particular steroids in arresting angiogenesis, in the presence of heparin fragments has been studied and reported by Crum et al., A New Class of Steroids Inhibits Angiogenesis in the presence of Heparin or a Heparin Fragment, Science, Vol. 230, 1375-1378, (1985). The article identifies the minimum essential structure of a steroid necessary to exhibit angiostatic properties. It is recognized that this steroid function is independent of glucocorticoid and mineralcorticoid activity.

It is, therefore, an object of the invention to provide a novel therapeutic composition for arresting angiogenesis, and cell, capillary or membrane leakage comprising a pharmaceutically active amount of angiostatic steroid and sodium pentosan polysulfate.

The molecular weight of the sodium pentosan polysulfate of the invention is preferably 1,600-6,000, or, more preferably, approximately 2,000. The pentosan polysulfate preferably also has substantially no anticoagulant properties.

The angiostatic steroid is selected from the group of: cortisone, hydrocortisone, and deltacortisone.

The composition may further include a buffering agent in an amount sufficient to maintain a pH of at

least about 8 to prevent deactivation of the antigiostatic steroid when exposed to an acidic environment in the body. The preferred buffering agent is sodium bicarbonate.

A three component composition may include pharmaceutically effective amounts of sodium pentosan polysulfate and angiostatic steroid, together with sufficient amounts of buffer.

The composition is adapted for oral administration. The preferred angiostatic steroid is deltacortisone, in combination with the sodium pentosan polysulfate, in pharmaceutically effective amounts.

The composition of the invention may be in non-aqueous powdered, liquid, or pill form. In this case, the preferred angiostatic steroids are those which are insoluble or have low water solubilities. The most preferred water insoluble angiostatic steroid is deltacortisone.

The inventive composition may be formulated in aqueous form, in which case the angiostatic steroid is preferably selected from the group consisting of cortisone and hydrocortisone. The aqueous composition may further include buffer, as noted above.

The composition of the invention has proven useful in treating bladder cancer and other diseases.

It has been discovered that combinations of certain steroids and sodium pentosan polysulfate, unexpectedly, when administered in pharmaceutically effective amounts, succeed in arresting angiogenesis, cell membrane leakage, and capillary leakage or exchange.

Cell membrane leakage has now, in tests conducted by Applicant, been discovered to be the cause of a number of different diseases whose basis was not previously understood. Thus, antibiotic-induced interstitial cystitis is a specific disease entity which presents with pelvic pain before and after voiding, frequency and nocturia in the absence of infection with an alkaline urine. Arthritic symptoms, spastic colon and low grade fevers were seen in 34% of patients suspected of having the disease. Three hundred patients were cystoscoped under anesthesia and demonstrated petechial hemorrhaging, glomerulation or linear striation after decompression of the bladder. Bladder biopsies demonstrated loss of the surface glycosaminoglycans layer (GAG) with resultant loss of cellular integrity of the urothelial cells.

In a disease such as this, whereas prior treatments had been ineffective, treatment aimed at arresting angiogenesis and/or membrane and capillary leakage have now been found to provide cure in many cases.

The composition of the invention has been found to provide surprisingly good results in providing relief and cure, without disturbing side effects which are anticipated when purified and highly toxic forms of heparin and steroids are used. For example, research by the inventor has now shown that the leaky cell theory also explains immunemediated angiogenesis, including immunological interstitial cystitis, chronic cystitis, trigonitis, urethritis, arthritis, diabetes, certain types of tumor growth, including transitional cell carcinoma of the bladder, angiofibromas, angiosarcoma,bladder cancer, renal cell carcinoma, cervical cancer, hemangiomas and other vascular lesions, inflammatory angiogenesis including DES (diethylstilbesterol) cervicitis, psoriasis, vaginosis, inflammatory interstitial cystitis and other inflammatory conditions.

In contradistinction to the disclosure of European Patent Application 0 114 589, the instant invention is specifically directed to the use of sodium pentosan polysulfate, which can be characterized as a linear anionic polyelectrolyte of the sulphated polyanion type. While similar compounds covalently bound with protein naturally exist in blood and tissue where they enhance various systems and control enzyme reactions by virtue of density and distribution of the electronegative charge, such heparins are heterogeneous with respect not only to their molecular weight but also to the distribution of active groups. It is a specific aim of the instant convention, therefore, to specifically utilize sodium pentosan polysulfate, which is chemically well defined with constant pharmacological potency.

The base units of the sodium pentosan polysulfate are pentoses, with a polymer chain being made of 1-4 linked beta-D-xylopyranose residues as is shown by the following structural fragment, which depicts hydroxy groups completely converted to the sulfate radicals and substituted with sodium:

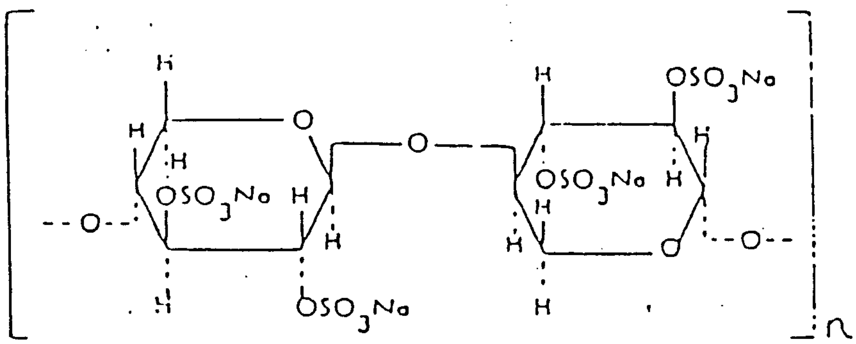

Because beta-glucosides do not show much natural tendency to form helices and the large number of negatively charged, mutually repelling $SO_4$ groups also tend to prevent coiling, this results in the polymer adopting a fairly rigid and linear configuration.

The molecular weight of the sodium pentosan polysulfate according to the invention is preferably between 1,600-6,000, and the sodium pentosan polysulfate preferably has no anticoagulant properties. A molecular weight composition of 2,000 is particularly preferred. One commercially available form of the sodium polyanion salt is $SP_{54}$, sold by BENE ARZNEIMITTEL GMBH, 8,000 Munich 71, Federal Republic of Germany.

When used alone, the sulphated polyanion exhibits significant thrombolytic, fibrinolytic and lipolytic action. Yet, unlike heparin, it is virtually free of any anticoagulant effect.

When used in combination with angiostatic steroids generally, the combination has proven unexpectedly useful in overcoming the effects of membrane leakage which have been the root cause of the diseases recited above.

The sodium pentosan polysulfate and steroid may be mixed together prior to administration or may be administered separately. The composition administration of the invention is administered orally, in any suitable form, including, but not limited to pill, powder, and aqueous form. The dosages administered are a function of the manner of administration.

The preferred concentration of sodium pentosan polysulfate for oral administration is 100-400 mg per 68kg (150 lbs) of body weight. The preferred concentration of angiostatic steroid is, generally, 100-200 mg; however, for deltacortisone, the preferred concentration is 1-100 mg, or, most preferably, 20 mg. The preferred concentration for hydrocortisone is 100 mg.

Although the above ranges have been provided as general guides, it is to be understood that dosage will vary as a function of body weight, tumor type and size, manner of administration, etc.

It is preferable that a buffering agent be incorporated in sufficient amounts so as to maintain a pH of 8 or above so as to prevent deactivation of the steroid. Amounts on the order of 50 milliequivalents are preferred.

A buffering agent used according to the invention is most preferably sodium bicarbonate, although other buffering agents such as magnesium, potassium, or calcium bicarbonate may also be used.

According to the invention the steroids used are angiostatic steroids. Anti-angiogenic or angiostatic activity is associated with the pregnane structure, and governed mainly by structural components on the D ring of the steroid. The 4,5 double bond in the A ring and the 11-hydroxyl on the C ring are not essential for angiostatic activity. Absence of the 17-hydroxyl and of carbons 20 and 21 on the D ring leads to successive reduction of angiostatic activity.

The preferred angiostatic steroids according to the invention are cortisone, hydrocortisone, and deltacortisone.

Deltacortisone can only be used in non-aqueous form since it is not water soluble to a sufficiently substantial extent. Deltacortisone has a half life of 8 to 10 hours such that it is far more effective when administered orally than steroids having shorter half lives.

For aqueous oral administration, angiostatic compounds used according to the invention must be of low water solubility. The preferred steroids for aqueous oral administration are cortisone and hydrocortisone.

Steroids generally have a low predictability, such that even analogues do not function predictably. Deltacortisone has not previously been disclosed for use in combination with heparin fragments generally, or pentosan polysulfate. A number of other steroids were tested, with heparin and heparin fragments, but were found ineffective in overcoming angiogenesis discussed above. These unsuccessful compounds included dexamethasone, corticosterone, desoxycorticosterone, progesterone, estrone, testosterone, and

cholesterol, pregnenolone, and cortexolone.

According to one embodiment of the invention, the mixture of the present invention may be administered from a container such as a bottle, having a first compartment and a second compartment separated by a breakable barrier such as a diaphragm. The first compartment contains a powdered angiostatic steroid of the invention and powdered pentosan polysulphate, while the second compartment contains a liquid mixture of buffering agent.

When the barrier is broken, such as by twisting and pushing the diaphragm down in the bottle, the contents of the first and second compartments are mixed and the steroid becomes activated by reason of the alkaline pH provided by the buffering agent. The actual way in which the components are divided is not critical except that the steroid and pentosan polysulphate must be separated from the buffer in the liquid state.

EXAMPLES

Example 1

Eighty-one patients exhibiting IgM vasculitis on bladder biopsies were orally administered the composition of the invention in liquid and/or pill at dosages of 200-400 mg/day $SP_{54}$ and 10 mg. deltacortisone twice a day every other day, for up to 11 months. About 76 responded extremely well to oral therapy, showing complete relief of symptoms. Ten patients were re-biopsied and there was no IgM staining present in those who responded. In those who did not respond IgM staining was identical to the original staining. Therapy could be reduced or stopped altogether in 18 patients with no recurrence after 5 months.

Example 2

100 mice injected with live transitional cell carcinoma subcutaneously, and were grown to a maximum size of 2 cm prior to therapy. The mice were given $SP_{54}$ and hydrocortisone orally and by injection. The composition was orally administered with $SP_{54}$ at 0.06 mg/ml and hydrocortisone at 0.5 mg/ml in drinking water. In others the composition was injected with 0.66 mg/ml $SP_{54}$ and hydrocortisone at 3 mg/ml twice daily, 0.55 ml. Both types of administration resulted in tumor regression. When $SP_{54}$ was eliminated from treatment the mice developed regression of tumor but were found to have lung metastasis which were not found in those mice treated with $SP_{54}$ and angiostatic steroid.

Example 3

A 55 year old male was found to have biopsy-proven angiosarcoma of the chest wall; the tumor doubling rate was 24 hours. The patient was started on 60 mg Prednisone daily and 800 mg $SP_{54}$ whereupon the tumor stopped doubling.

## Claims

1. An angiostatic composition for oral administration comprising a pharmaceutically effective amount of angiostatic steroid and sodium pentosan polysulfate.

2. The composition as defined by claim 1 wherein said sodium pentosan polysulfate has a molecular weight of 1600-6000.

3. The composition as defined by claim 2 wherein said sodium pentosan polysulfate has a molecular weight of about 2000.

4. The composition as defined by claim 1 further comprising a buffering agent in an amount sufficient to maintain a pH of at least 8 to prevent deactivation of said angiostatic steroid when exposed to an acidic environment in the body.

5. The composition as defined by claim 4 wherein said buffering agent is sodium bicarbonate.

6. The composition as defined by claim 1 wherein said angiostatic steroid is selected from: cortisone, hydrocortisone, and deltacortisone.

7. The composition as claimed in any one of claims 1-5 in non-aqueous form.

8. The composition as defined by claim 7 wherein said angiostatic steroid is deltacortisone.

9. The composition as defined by claim 1 or claim 7 wherein said sodium pentosan polysulfate comprises at least one uronic acid unit.

**Patentansprüche**

1. Angiostatische Zusammensetzung zur oralen Anwendung, welche eine pharmazeutisch wirksame Menge eines angiostatischen Steroid und Natriumpentosanpolysulfat enthält.

2. Zusammensetzung nach Anspruch 1, bei der das Natriumpentosanpolysulfat ein Molekulargewicht von 1600-6000 hat.

3. Zusammensetzung nach Anspruch 2, bei der das Natriumpentosanpolysulfat ein Molekulargewicht von ungefähr 2000 hat.

4. Zusammensetzung nach Anspruch 1, welche weiter ein Puffermittel enthält, dessen Menge groß genug ist einen pH-Wert von mindestens 8 einzustellen, um die Deaktivierung des angiostatischen Steroids zu verhindern, wenn es der sauren Umgebung des Körpers ausgesetzt wird.

5. Zusammensetzung nach Anspruch 4, bei der das Puffermittel Natriumbicarbonat ist.

6. Zusammensetzung nach Anspruch 1, bei der das angiostatische Steroid Cortison, Hydrocortison oder Deltacortison ist.

7. Zusammensetzung nach einem der Ansprüche 1 bis 5, welche in nichtwässriger Form vorliegt.

8. Zusammensetzung nach Anspruch 7, bei der das angiostatische Steroid Deltacortison ist.

9. Zusammensetzung nach Anspruch 1 oder 7, bei der das Natriumpentosanpolysulfat mindestens eine Uronsäureeinheit enthält.

**Revendications**

1. Composition angiostatique pour l'administration orale comprenant une quantité pharmaceutiquement efficace d'un stéroïde angiostatique et de pentosane polysulfate de sodium.

2. Composition selon la revendication 1, dans laquelle ledit pentosane polysulfate de sodium a une masse moléculaire de 1600-6000.

3. Composition selon la revendication 2, dans laquelle ledit pentosane polysulfate de sodium a une masse moléculaire d'environ 2000.

4. Composition selon la revendication 1, comprenant en outre un agent tampon dans une quantité suffisante pour maintenir un pH d'au moins 8 pour empêcher la désactivation dudit stéroïde angiostatique lorsqu'il est exposé à un environnement acide dans l'organisme.

5. Composition selon la revendication 4, dans laquelle ledit agent tampon est du bicarbonate de sodium.

6. Composition selon la revendication 1, dans laquelle ledit stéroïde angiostatique est choisi parmi la cortisone, l'hydrocortisone et la deltacortisone.

7. Composition selon l'une quelconque des revendications 1 à 5, sous forme non aqueuse.

8. Composition selon la revendication 7, dans laquelle ledit stéroïde angiostatique est la deltacortisone.

9. Composition selon la revendication 1 ou 7, dans laquelle ledit pentosane polysulfate de sodium comprend au moins un motif acide uronique.